# EUROPÄISCHE PATENTSCHRIFT

(11) **EP 1 078 932 B1**
(45) Veröffentlichungstag und Bekanntmachung des Hinweises auf die Patenterteilung: **17.03.2004**
(21) Anmeldenummer: 00115122.4
(22) Anmeldetag: 12.07.2000
(51) Int. Cl.: C07H 7/027

(54) **Verfahren zur Herstellung von 2-Keto-L-gulonsäureestern**
Process for the preparation of 2-keto-2-gulonic acid esters
Procédé de préparation d'esters de l'acide céto-2-L-gulonique

(30) Priorität: 19.08.1999 DE 19938980
(43) Veröffentlichungstag der Anmeldung: 28.02.2001
(73) Patentinhaber: BASF AKTIENGESELLSCHAFT, 67056 Ludwigshafen (DE)
(72) Erfinder: Böttcher, Andreas, Dr., 69226 Nussloch (DE); Burst, Wolfram, 68199 Mannheim (DE)

(56) Entgegenhaltungen:
- EP-A- 0 535 927
- EP-A- 0 671 405
- WO-A-97/43433
- DD-A- 39 696
- DE-A- 19 829 809

## Beschreibung

Die Erfindung betrifft ein neues Verfahren zur Herstellung von 2-Keto-L-gulonsäureestern. Diese Ester stellen wichtige Zwischenprodukte für die Synthese von L-Ascorbinsäure (Vitamin C) dar.

Die Veresterung von 2-Keto-L-gulonsäure mit einem niederen Alkohol, insbesondere Methanol, ist aus zahlreichen Publikationen bekannt.

Grundsätzlich wird bei diesem Reaktionstyp die Ausbeute durch das Veresterungsgleichgewicht limitiert. Die Veresterung profitiert dabei von einem Alkohol-Überschuß oder von einem kontinuierlichen Entfernen des gebildeten Wassers, das beispielsweise durch azeotrope Destillation erfolgen kann.

Bei den üblichen Verfahren, u.a. dem Reichstein Verfahren, wird zur Erzielung eines hohen Veresterungsgrades und damit einer befriedigenden Ausbeute üblicherweise eine lange Kochzeit gewählt. Diese wirkt sich jedoch zu Lasten der Reinheit aus, da sowohl die 2-Keto-L-gulonsäure als auch ihr Methylester unter diesen Bedingungen weitere Nebenprodukte bilden können.

Um den Wassergehalt bei der Veresterung zu reduzieren, sind verschiedene Methoden vorgeschlagen worden. Wasser kann während der Reaktion durch fortwährendes Abdestillieren des Wasser/Alkoholgemisches, Behandeln des Destillates mit Molekularsieben und Rezyklisierung des so getrockneten Alkohols entfernt werden (Pol. Pat. 57042; Pol. Pat. 57573). Als eine andere Möglichkeit wurde das während der Reaktion fortwährende Abdestillieren des Wasser/Alkoholgemisches und Ersatz mit frischem trockenem Alkohol erwähnt (EP-A-0 535 927). Dabei muß in beiden Fällen eine große Menge Alkohol mit entsprechend großem Energieaufwand abdestilliert werden. Außerdem werden Reaktionszeiten von bis zu 10 Stunden notwendig, mit der Gefahr von Zersetzung und Nebenreaktionen.

EP-A-0 671 405 beschreibt ein Verfahren zur Herstellung von 2-Keto-L-gulonsäure-methyl- oder -ethylester durch Veresterung von 2-Keto-L-gulonsäure mit Methanol bzw. Ethanol in Gegenwart eines sauren Ionenaustauschers. Die Reaktion erfolgt dabei in einem mit Ionenaustauscherharz gefüllten Rohrreaktor bei einer mittleren Verweilzeit der Reaktanden zwischen 10 und 120 Minuten.

EP-A-0 403 993 beschreibt ein Verfahren zur Herstellung von 2-Keto-L-gulonsäure-methylester, bei der die Veresterung nur partiell, d.h. nicht bis zur Erreichung des Veresterungsgleichgewichts durchgeführt wird. In einer Zwischenstufe werden durch Zugabe von Natriumbicarbonat bzw. von Kaliumbicarbonat - in einer im wesentlichen zur Neutralisation der Esterlösung gerade ausreichenden Menge - nicht veresterte 2-Keto-L-gulonsäure sowie vorhandene Verunreinigungen ausgefällt und abgetrennt.

In einem gemäß WO 99/03853 beschriebenen Verfahren wird 2-Keto-L-gulonsäure in einem zweistufigen Veresterungsprozeß zu 2-Keto-L-gulonsäureester umgesetzt, wobei die nach dem ersten Veresterungsschritt entstehende Lösung zur Entfernung des gebildeten Reaktionswassers wenigstens teilweise eingedampft und der entstandene Rückstand einem zweiten Veresterungsprozeß unterzogen wird.

WO 97/43433 sowie US 5,391,770 beschreiben die Synthese von 2-Keto-L-gulonsäure-butylester durch mehrstündiges Rückflußkochen von 2-Keto-L-gulonsäure in Butanol in Gegenwart von p-Toluolsulfonsäure und anschließendes Auskristallisieren des Wertproduktes durch Abkühlen der Reaktionsmischung.

DE-A-198 29 809 betrifft ein Verfahren zur Herstellung von Estern aus Alkohol und Carbonsäure mittels eines Katalysators und Abtrennung des Esters in einer mit Einbauten versehenen Rektifikationskolonne.

Für Diaceton-2-keto-L-gulonsäure (DAKS), dem Reaktionsprodukt aus dem klassischen Reichstein-Verfahren [Reichstein und Grüssner, Helv. Chim. Acta 17, 311 (1934)] ist in der US-Patentschrift 2,491,933 eine Veresterung unter Abspaltung von Aceton erwähnt. Das gebildete Aceton wird über Kopf der Destillationskolonne als Leichtsieder entfernt.

Die DD-39 696 beschreibt ein Verfahren zur Herstellung von Keto-2-Lgulonsäuremethylester ausgehend von Diaceton-keto-2-L-gulonsäure, das dadurch gekennzeichnet ist, daß Reaktionswasser zumindest teilweise mit dem überschüssigen Methanol aus der Reaktionslösung entfernt wird.

Die oben genannten Veresterungsmethoden sind darüberhinaus nur bedingt für eine kontinuierliche Arbeitsweise geeignet. Lediglich für den Methyl- und den Ethylester der 2-Keto-L-gulonsäure wurden Methoden für eine kontinuierliche Arbeitsweise beschrieben. Auch unter optimierten Bedingungen sind bei all diesen Reaktionen mittlere Verweilzeiten von mehr als 10 min. erforderlich. Zusätzlich wird im Reaktionsgemisch immer noch ein zu hoher Restwassergehalt gemessen. Dieser ist bei einigen Folgereaktionen des Esters, z.B. für eine Vitamin C Synthese meist nicht erwünscht. Es bestand daher die Aufgabe, ein Verfahren zur Herstellung von 2-Keto-L-gulonsäureestern bereitzustellen, welches die oben genannten Nachteile nicht aufweist.

Diese Aufgabe wurde gelöst durch ein Verfahren zur Herstellung von 2-Keto-L-gulonsäure-C₁-C₁₀-alkylester durch Veresterung von 2-Keto-L-gulonsäure oder Diaceton-2-keto-L-gulonsäure mit einem C₁-C₁₀-Alkohol in Gegenwart eines sauren Katalysators, dadurch gekennzeichnet, daß man die Veresterung in einem Flüssigkeitsfilm an einer heißen Oberfläche bei gleichzeitiger Abtrennung von Wasser durchführt.

In der obigen Definition des Erfindungsgegenstandes umfasst der Begriff "Flüssigkeitsfilm" die von der Reaktionsmischung gebildeten dünnen flüssigen oder fließfähigen Filme bzw. Schichten mit einer Dicke im Bereich von 0,05 bis 10 mm, bevorzugt 0,05 bis 5 mm, besonders bevorzugt im Bereich von 0,1 bis 2 mm. Solche dünnen Schichten werden beispielsweise durch Abrieselnlassen der flüssigen Reaktionsmischung, Einwirkung von Zentrifugalkraft oder durch besonders konstruierte Wischer auf den beheizten Flächen erzeugt. Typische Apparate zur Erzeugung solcher dünnen Schichten sind u.a. Fallfilm- oder Fall strom-Verdampfer, Sambay- und Luwa-Dünnschicht-Verdampfer [= Ver dampfer, bei denen mittels rotierender Wischblätter oder Rollen dünne Flüssigkeitsfilme erzeugt werden], Rotations-Verdampfer aber auch Dünnschicht-Rektifikationskolonnen [z.B. Füllkörperund Filmkolonnen, Rektifikatoren mit rotierenden Einsätzen (Sprünkolonnen)]. Eine nähere Beschreibung der hier genannten Reaktoren findet sich in Ullmanns Encyklopädie der technischen Chemie, 4. Auflage, Band 2 (1972), S. 516, 533-537, 652-660 sowie in Band 3 (1973), S. 386-388.

Zur Veresterung eignen sich prinzipiell alle C₁-C₁₀-Alkohole, vorteilhafterweise gesättigte, verzweigte oder unverzweigte Alkylalkohole mit einer Kohlenstoffanzahl größer gleich 3, bevorzugt Alkohole mit einem Alkylrest von 3 bis 10 Kohlenstoffatomen, beispielsweise n-Propanol, Isopropanol, 1-Butanol, 2-Butanol, 2-Methyl-1-propanol, 2-Methyl-2-propanol, 1-Pentanol, 2-Pentanol, 3-Pentanol, 1-Hexanol, 2-Hexanol, 1-Heptanol, 2-Heptanol, 1-Octanol, 2-Octanol, 3-Octanol, 1-Nonanol, 2-Nonanol, 1-Decanol, 2-Decanol, 4-Decanol, besonders bevorzugt C₃-C₈-Alkohole, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol. Ganz besonders bevorzugte Alkohole sind n-Butanol und n-Pentanol.

Der Alkohol wird dabei in einem 2- bis 10-fachen, bevorzugt 3- bis 8-fachen, besonders bevorzugt 4- bis 6-fachen molaren Überschuß, bezogen auf die eingesetzte 2-Keto-L-gulonsäure bzw. Diaceton-2-keto-L-gulonsäure eingesetzt.

Im Verlauf der Veresterungsreaktion kann sowohl das als Lösungsmittel mitgeführte als auch das bei der Veresterung zusätzlich gebildete Wasser als leichter siedendes Azeotrop aus dem Reaktionsraum über die Gasphase entfernt werden.

Falls erforderlich kann nach der Kondensation eine Phasentrennung (Alkohol/Wasser) mit Rückführung des Veresterungsalkohols erfolgen. In der Regel ist dabei eine vollständige Entwässerung des rückgeführten Alkohols, z.B. durch Membranverfahren oder durch Destillation, jedoch nicht erforderlich, da erfindungsgemäß eine vollständige Entwässerung im Reaktionsraum erfolgt.

Bei der Veresterung von 2-Keto-L-gulonsäure mit C₁-C₃-Alkoholen kann es aufgrund der niedrigen Siedepunkte dieser Alkohole bei der destillativen Wasserabtrennung zu unerwünschten Verlusten an Alkoholen und damit zu Ausbeuteverlusten kommen. Durch entsprechende Ergänzung der Alkoholverluste während der Reaktion läßt sich die Veresterungsrate wieder steigern.

Durch Zusatz eines sauren Katalysators wird die Veresterungsreaktion in an sich bekannter Weise katalysiert. Der Katalysator wird dabei in Mengen von 0,001 bis 0,2 Mol, bevorzugt in Mengen von 0,005 bis 0,1 Mol, besonders bevorzugt 0,005 bis 0,05 Mol pro Mol 2-Keto-L-gulonsäure bzw. Diaceton-2-keto-L-gulonsäure eingesetzt.

Als Veresterungskatalysatoren können in der Regel alle an sich bekannten homogenen oder heterogenen sauren Katalysatoren eingesetzt werden.

Als homogene Katalysatoren sind beispielsweise Mineralsäuren bzw. deren Ester geeignet. Dazu zählen insbesondere Phosphorsäure, Phosphorsäure-monobutylester, Phosphorsäure-dibutylester, Phosphorsäure-monopentylester, Phosphorsäure-dipentylester, Schwefelsäure, Schwefelsäure-monobutylester, Schwefelsäure-monopentylester, Chlorwasserstoff, p-Toluolsulfonsäure, Methansulfonsäure, Trifluormethansulfonsäure, Chlorsulfonsäure und Trifluoressigsäure. Auch 2-Keto-L-gulonsäure, Diaceton-2-keto-L-gulonsäure oder Ascorbinsäure können als Veresterungskatalysatoren eingesetzt werden.

Bevorzugt verwendet man Schwefelsäure, p-Toluolsulfonsäure, Methansulfonsäure oder Monoalkylsulfate der verwendeten Alkohole. Die Monoalkylsulfate spalten bei Temperaturen oberhalb von 70°C Schwefelsäure ab [Popelier, Bull. Soc. Chim. Belg. 35, 265 (1926)], die dann katalytisch wirkt. Besonders bevorzugte homogene saure Katalysatoren sind Schwefelsäure und p-Toluolsulfonsäure.

Im Falle der o.g. homogenen Katalysatoren können diese entweder einem der Edukte vor der Reaktion zugemischt werden oder als separater Strom in den Reaktor eingespeißt werden.

Heterogene Katalysatoren sind vorteilhafterweise im Reaktor in der heißen Reaktionszone fixiert. Für den Einbau heterogener Katalysatoren in Destillationskolonnen sind in der Literatur zahlreiche Konstruktionsmöglichkeiten beschrieben. Hierzu gehören Verweilzeitböden, bei denen der Katalysator auf den Böden oder in deren Ablaufschächten angeordnet sein kann, ferner beschichtete Füllkörper, gewickelte und strukturierte Packungen mit eingewebtem Katalysator.

Als heterogene Katalysatoren kommen die an sich bekannten sauren Katalysatoren, bevorzugt saure Ionentauscher als auch Zeolithe, in Frage.

Unter der Bezeichnung "saure Kationenaustauscher" sind kommerziell erhältliche Harze bzw. Detoxane zu verstehen, wie z.B. Lewatit® S 100, SP 112 oder Lewatit® 2631 (Bayer) oder Amberlite® 18 und IRA 120 oder Amberlyst® 15 oder Duolite® C 20, C 26 und C 264 (Rohm & Haas) oder Dowex® Ionentauscher.

Zeolithe sind kristalline Aluminosilikate, die eine hochgeordnete Struktur mit einem starren dreidimensionalen Netzwerk von SiO₄oder AlO₄-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffatoms ausgeglichen. Ein Kationenaustausch ist daher möglich. Die Räume zwischen den Tetraedern sind vor der Dehydration durch Trocknen bzw. Calcinieren von Wassermolekülen besetzt.

Geeignete Zeolithe sind z.B. solche vom Pentasil-Typ, besonders Aluminosilikat-Zeolithe oder Borosilikat-Zeolithe. Durch partielle Verkokung (pre-coke) ist es möglich, die Aktivität des Katalysators für ein Selektivitätsoptimum des gewünschten Reaktionsproduktes einzustellen.

Die Veresterung wird erfindungsgemäß in einem dünnen Flüssigkeitsfilm an heißen Oberflächen durchgeführt, wobei die Temperatur der Oberfläche im Bereich von 50 bis 250°C, bevorzugt von 80 bis 200°C, besonders bevorzugt im Bereich von 90 bis 150°C liegt.

Die Verweilzeit des Reaktionsgemisches an der heißen Oberfläche liegt im Bereich von 1 bis 400 Sekunden, bevorzugt 20 bis 300 Sekunden, besonders bevorzugt im Bereich von 30 bis 250 Sekunden.

Die Reaktion erfolgt erfindungsgemäß im Druckbereich zwischen 1 und 1000 mbar und wird bevorzugt zwischen 50 und 1000 mbar, besonders bevorzugt zwischen 500 und 950 mbar ausgeführt.

vorzugsweise werden die eingangs genannten Reaktoren so dimensioniert, daß eine vollständige Entfernung des Reaktionswassers und damit eine vollständige Veresterung bei kurzen Verweilzeiten erzielt wird. Damit wird gewährleistet, daß Folgereaktionen des Reaktionsproduktes, z.B. mit dem Veresterungskatalysator vermieden werden. Als Apparate eignen sich bevorzugt die an sich bekannten u.a. in Chem. Ing. Techn. 43, 1101 (1971) beschriebenen Reaktionskolonnen mit Abtriebs- und Verstärkungsteil sowie Dünnschich-t-Verdampfer oder Fallstrom-Verdampfer.

Diese Art der Reaktionsführung ist für Ester der 2-Keto-L-gulonsäure, die nicht spontan kristallisieren, z.B. 2-Keto-L-gulonsäure-butylester, besonders gut geeignet.

Neben 2-Keto-L-gulonsäure kann auch Diaceton-2-keto-L-gulonsäure unter den oben genannten Bedingungen in gleicher Weise verestert werden. Dabei erfolgt zusätzlich eine Abspaltung der Aceton-Schutzgruppen. Für die Abspaltung werden zwei Moläquivalente Wasser benötigt, während gleichzeitig ein Moläquivalent Wasser bei der Veresterungsreaktion gebildet wird. In einfachster Weise setzt man daher das Monohydrat der Diaceton-2-keto-L-gulonsäure ein. Die Reaktion erfolgt im gleichen, wie oben beschriebenen Druck- und Temperaturbereich. Das gebildete Aceton wird als Leichtsieder während der Veresterungsreaktion zu Beginn bzw. zusammen mit überschüssigem, wasserhaltigem Lösungsmittel abdestilliert und kann nach Isolierung und Reingewinnung zurückgeführt und beispielsweise zur Synthese von Diaceton-2-keto-L-gulonsäure nach dem klassischen Reichstein-Verfahren wiederverwendet werden. Bei Einsatz von wasserfreier Diaceton-2-keto-L-gulonsäure muß zusätzlich 1 mol Wasser zugesetzt werden.
Für das erfindungsgemäße Verfahren wird bevorzugt 2-Keto-L-gulonsäure als Ausgangsmaterial eingesetzt. Die Säure kann dabei sowohl in kristalliner Form, beispielsweise als getrocknetes oder schleuderfeuchtes Monohydrat, als wasserfreie Verbindung als auch als wäßrige Lösung, beispielsweise als aufkonzentrierte Fermentationslösung eingesetzt werden.

Das Monohydrat der 2-Keto-L-gulonsäure fällt in der Regel bei der Kristallisation aus Wasser oder wasserhaltigen, organischen Lösungsmitteln an. Durch Abschleudern der Kristallmaische ist feuchtes Monohydrat zugänglich. Dieses kann als schleuderfeuchtes Produkt direkt in der erfindungsgemäßen Veresterungsreaktion eingesetzt oder unter milden Bedingungen getrocknet werden.

Vorteilhafterweise kann man bei dem erfindungsgemäßen Verfahren auf die Trocknung bzw. Entwässerung des Monohydrates der 2-Keto-L-gulonsäure verzichten, da bei der nachfolgenden, erfindungsgemäßen Aktivierungsreaktion ohnehin eine azeotrope Entwässerung durchgeführt wird.

Der Umsatzgrad der 2-Keto-L-gulonsäure bei der Veresterungsreaktion liegt im erfindungsgemäßen Verfahren deutlich über 90 %, vorzugsweise in einem Bereich von 95 bis 99 %.

Das Reaktionsgemisch erstarrt beim Abkühlen in einem Temperaturbereich von 30 bis 70°C. Beim Erwärmen bildet sich die Schmelze reversibel und ohne Zersetzung zurück.

Die Veresterungsreaktion von 2-Keto-L-gulonsäure bzw. Diaceton-2-keto-L-gulonsäure kann diskontinuierlich oder bevorzugt kontinuierlich betrieben werden. Auch unter diesen Bedingungen entsteht am Ende der Veresterung eine schmelzflüssige Form des entsprechenden 2-Keto-L-gulonsäure-alkylesters.

Eine besonders bevorzugte Ausführungsform des erfindungsgemäßen Verfahrens ist dadurch gekennzeichnet, daß man in einem Dünnschicht-Verdampfer oder in einer Rektifikationskolonne eine wäßrige Lösung von 2-Keto-L-gulonsäure kontinuierlich mit n-Butanol in Gegenwart von Schwefelsäure an einer 90 bis 150°C heißen Oberfläche im Druckbereich zwischen 500 und 950 mbar und einer Verweilzeit von 30 bis 250 Sekunden verestert.

Der gebildete 2-Keto-L-gulonsäure-alkylester läßt sich sowohl unter sauren als auch unter basischen Bedingungen in an sich bekannter Weise in L-Ascorbinsäure überführen.

Das erfindungsgemäße Verfahren zeichnet sich durch folgende Vorteile aus:

Die Veresterungsreaktion kann sowohl batchweise als auch vorteilhafterweise kontinuierlich betrieben werden und führt insbesondere bei der Verwendung von Alkoholen mit einer Kohlenstoffanzahl größer 3 zu keinen wesentlichen Verlusten an Alkoholen bei der destillativen Abtrennung von Wasser.

Die Verweilzeiten sind im Vergleich zu den literaturbekannten Verfahren zur Herstellung von 2-Keto-L-gulonsäureestern sehr kurz, so daß in der Regel Apparate mit kleinen Dimensionen eingesetzt werden können.

Das Reaktionsprodukt, beispielsweise der 2-Keto-L-gulonsäure-butylester, kann entweder unmittelbar direkt nach gängigen Verfahren isoliert oder ohne weitere Isolierung für Folgereaktionen verwendet werden.

Die Reaktionsparameter wie Temperatur und Zulaufgeschwindigkeit der Edukte während der Veresterung können so eingestellt werden, daß der Wassergehalt und/oder der Anteil des Veresterungsalkohols im Reaktionsgemisch gegen Null geht.

Überraschend wurde nun gefunden, daß bei der erfindungsgemäßen Veresterungsreaktion in den Reaktoren keine Feststoffbildung auftritt, obwohl die Keto-L-gulonsäure in niederen Alkoholen, wie z.B. Methanol, Ethanol, Propanol, Butanol oder Pentanol, schwer löslich ist und die gebildeten Ester nach der Wasserabtrennung lösungsmittelfrei vorliegen.

Anhand der folgenden Beispiele soll das erfindungsgemäße Verfahren näher erläutert werden.

### Beispiel 1

### Veresterung im Dünnschicht-Verdampfer

Die für die Veresterung verwendete Versuchsapparatur bestand aus einem Dünnschicht-Verdampfer aus Glas mit einer Verdampferfläche von 0,046 m², je einer Vorlage für 2-Keto-L-gulonsäure und n-Butanol, einer Mischerstrecke für den Zusatz katalytischer Mengen Schwefelsäure im Zulaufstrom sowie einer Destillat- und einer Schwersiedervorlage.

Mit einer Geschwindigkeit von 12 g/Std. wurde eine wäßrige 2-Keto-L-gulonsäurelösung zusammen mit 660 g/Std. n-Butanol und einer katalytischen Menge konzentrierter Schwefelsäure kontinuierlich am Kopf des Verdampfers zugefahren. Bei einem Druck von 900 mbar stellte sich im Verdampfer eine Innentemperatur von 110°C ein. Die mittlere Verweilzeit des Reaktionsgemisches betrug ca. 100 Sekunden. Nach Einstellung des stationären Zustandes wurden 10 g/Std. eines schwach gelb gefärbten, viskosen Öls im Sumpf abgezogen. Das Reaktionsprodukt, das aus 97 Gew.-% 2-Keto-L-gulonsäure-n-butylester, 0,5 Gew.-% 2-Keto-L-gulonsäure und 2,5 Gew.-% n-Butanol bestand, erstarrte bei einer Temperatur von 65°C zu einer festen, amorphen Masse.

Durch Veränderung der mittleren Verweilzeit, der Zulaufmenge und der Temperaturdifferenz in der Reaktionszone an der Verdampferwandung konnte die Zusammensetzung des Produktgemisches zugunsten des Wertproduktes weiter verbessert werden.

### Beispiel 2

### Veresterung in einer Reaktionskolonne ohne Reaktiveinbauten

Die Reaktionskolonne mit einem Innendurchmesser von 0,044 m bestand aus einem Verstärkungs- und Abtriebsteil von je 1 m Länge.

Bei einer Zusammensetzung des Zulaufes, analog Beispiel 1, stellte sich bei einem Druck von 900 mbar eine Verdampf ertemperatur von 112°C ein. Das Reaktionsprodukt bestand aus 98 % 2-Keto-L-gulonsäure-n-butylester, 0,1 % 2-Keto-L-gulonsäure und 1,9 % n-Butanol. Es wurde in einer 70°C heißen Vorlage gesammelt und anschließend auf einer Schuppenwalze an einer gekühlten Oberfläche geschuppt.

### Beispiel 3

### Veresterung in einer Reaktionskolonne mit Reaktivteil

Die Veresterung wurde in einer Laborkolonne mit 0,055 m Durchmesser durchgeführt. Verstärkungs- und Abtriebteil wurden durch je 4 Packungsabschnitte von jeweils max. 0,5 bis 1 m à 300 m²/m³ realisiert. Den Reaktivteil bildete eine Katalysatorpackung mit einem Veresterungskatalysator (Ionentauscher, Detoxane, Lewatit® 2631). Die Apparatur wurde zwischen 500 und 950 mbar betrieben. Der Druckverlust lag im Reaktivteil bei 30 mbar.

Im stationären Zustand betrug der Umsatz 99,2 %. Der Restwassergehalt lag bei < 50 ppm. Daneben waren geringe Mengen an n-Butanol nachzuweisen.

## Patentansprüche

1. Verfahren zur Herstellung von 2-Keto-L-gulonsäure-C₁-C₁₀-alkylester durch Veresterung von 2-Keto-L-gulonsäure oder Diaceton-2-keto-L-gulonsäure mit einem C₁-C₁₀-Alkohol in Gegenwart eines sauren Katalysators, **dadurch gekennzeichnet, daß** man die Veresterung in einem Flüssigkeitsfilm an einer heißen Oberfläche bei gleichzeitiger Abtrennung von Wasser durchführt.

2. Verfahren nach Anspruch 1, **dadurch gekennzeichnet, daß** man die Veresterung in einem Dünnschicht-Verdampfer oder in einer Rektifikationskolonne durchführt.

3. Verfahren nach einem der Ansprüche 1 oder 2, **dadurch gekennzeichnet, daß** man das Wasser destillativ abtrennt.

4. Verfahren nach einem der Ansprüche 1 bis 3, **dadurch gekennzeichnet, daß** die Verweilzeit der Reaktionslösung an der heißen Oberfläche 1 bis 400 Sekunden beträgt.

5. Verfahren nach einem der Ansprüche 1 bis 4, **dadurch gekennzeichnet, daß** man die Veresterung im Druckbereich zwischen 1 und 1000 mbar durchführt.

6. Verfahren nach einem der Ansprüche 1 bis 5, **dadurch gekennzeichnet, daß** man die Veresterung an einer 50 bis 250°C heißen Oberfläche durchführt.

7. Verfahren nach einem der Ansprüche 1 bis 6, **dadurch gekennzeichnet, daß** man die Veresterung kontinuierlich durchführt.

8. Verfahren nach einem der Ansprüche 1 bis 7, **dadurch gekennzeichnet, daß** man die Veresterung mit einem C₃-C₈-Alkohol, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol durchführt.

9. Verfahren nach einem der Ansprüche 1 bis 8, **dadurch gekennzeichnet, daß** man die Veresterung in Gegenwart einer Mineralsäure, eines sauren Ionenaustauscherharzes oder saurer Zeolithe durchführt.

10. Verfahren nach einem der Ansprüche 1 bis 9, **dadurch gekennzeichnet, daß** man in einem Dünnschicht-Verdampfer oder in einer Rektifikationskolonne eine wäßrige Lösung von 2-Keto-L-gulonsäure kontinuierlich mit n-Butanol in Gegenwart von Schwefelsäure an einer 90 bis 150°C heißen Oberfläche im Druckbereich zwischen 500 und 950 mbar und einer Verweilzeit von 30 bis 250 Sekunden verestert.

## Claims

1. A process for the preparation of C₁-C₁₀-alkyl 2-keto-L-gulonates by esterifying 2-keto-L-gulonic acid or diacetone-2-keto-L-gulonic acid with a C₁-C₁₀-alcohol in the presence of an acidic catalyst, wherein the esterification is carried out in a liquid film on a hot surface with simultaneous removal of water.

2. A process as claimed in claim 1, wherein the esterification is carried out in a thin-film evaporator or in a rectification column.

3. A process as claimed in claim 1 or 2, wherein the water is removed by distillation.

4. A process as claimed in any of claims 1 to 3, wherein the residence time of the reaction solution on the hot surface is from 1 to 400 seconds.

5. A process as claimed in any of claims 1 to 4, wherein the esterification is carried out at from 1 to 1,000 mbar.

6. A process as claimed in any of claims 1 to 5, wherein the esterification is carried out on a hot surface at from 50 to 250°C.

7. A process as claimed in any of claims 1 to 6, wherein the esterification is carried out continuously.

8. A process as claimed in any of claims 1 to 7, when the esterification is carried out using a C₃-C₈-alcohol selected from the group consisting of n-propanol, isopropanol, 1-butanol, 2-butanol, 2-methyl-1-propanol, 2-methyl-2-propanol, 1-pentanol, 1-hexanol and 1-octanol.

9. A process as claimed in any of claims 1 to 8, wherein the esterification is carried out in the presence of a mineral acid, of an acidic ion exchange resin or an acidic zeolite.

10. A process as claimed in any of claims 1 to 9, wherein an aqueous solution of 2-keto-L-gulonic acid is esterified continuously with n-butanol in the presence of sulfuric acid on a hot surface at from 90 to 150°C and from 500 to 950 mbar and in a residence time of from 30 to 250 seconds in a thin-film evaporator or in a rectification column.

## Revendications

1. Procédé pour la préparation des esters alkyliques en C1-C10 de l'acide 2-céto-L-gulonique par estérification de l'acide 2-céto-L-gulonique ou de l'acide diacétone-2-céto-L-gulonique à l'aide d'un alcool en C1-C10 en présence d'un catalyseur acide, **caractérisé en ce que** l'on procède à l'estérification dans une pellicule de liquide sur une surface chaude avec séparation simultanée de l'eau.

2. Procédé selon la revendication 1, **caractérisé en ce que** l'on procède à l'estérification dans un évaporateur à couche mince ou dans une colonne de rectification.

3. Procédé selon l'une des revendications 1 ou 2, **caractérisé en ce que** l'on sépare l'eau par distillation.

4. Procédé selon une des revendications 1 à 3, **caractérisé en ce que** la durée de passage de la solution de réaction sur la surface chaude est de 1 à 400 s.

5. Procédé selon une des revendications 1 à 4, **caractérisé en ce que** l'estérification est réalisée dans un intervalle de pression de 1 à 1000 mbar.

6. Procédé selon une des revendications 1 à 5, **caractérisé en ce que** l'estérification est réalisée sur une surface à une température de 50 à 250°C.

7. Procédé selon une des revendications 1 à 6, **caractérisé en ce que** l'on réalise l'estérification en continu.

8. Procédé selon une des revendications 1 à 7, **caractérisé en ce que** l'estérification est réalisée à l'aide d'un alcool en C3-C8 choisi dans le groupe consistant en le n-propanol, l'isopropanol, le 1-butanol, le 2-butanol, le 2-méthyl-1-propanol, le 2-méthyl-2-propanol, le 1-propanol, le 1-hexanol et le 1-octanol.

9. Procédé selon l'une des revendications 1 à 8, **caractérisé en ce que** l'estérification est réalisée en présence d'un acide minéral, d'une résine échangeuse d'ions acide et d'une zéolithe acide.

10. Procédé selon l'une des revendications 1 à 9, **caractérisé en ce que** l'on estérifie dans un évaporateur à couche mince ou dans une colonne de rectification une solution aqueuse de l'acide 2-céto-L-gulonique en continu par le n-butanol en présence d'acide sulfurique sur une surface à une température de 90 à 150°C dans l'intervalle de pression de 500 à 960 mbar et avec une durée de passage de 30 à 250 s.
